# EUROPEAN PATENT SPECIFICATION

(11) **EP 3 141 227 B1**
(45) Date of publication and mention of the grant of the patent: **11.03.2020**
(21) Application number: 16002126.7
(22) Date of filing: 04.01.2011
(51) Int. Cl.: A61F 9/007, A61F 9/008

(54) **AN OPHTHALMIC SURGICAL DEVICE**
AUGENCHIRURGIEVORRICHTUNG
DISPOSITIF CHIRURGICAL OPHTALMIQUE

(30) Priority: 04.01.2010 NL 2004047
(43) Date of publication of application: 15.03.2017
(62) Divisional of application: 11701869.7
(73) Proprietor: Ellex iScience, Inc., Freemont, CA 94538 (US)
(72) Inventor: Vijfvinkel, Jan Gerit, 3214 VN Zuidland (NL)
(74) Representative: Sattler de Sousa e Brito, Clara

(56) References cited:
- WO-A2-2005/070490
- WO-A2-2006/066103
- US-A- 5 514 125
- US-A1- 2002 111 608
- US-B1- 6 241 721

## Description

The invention relates to an ophthalmic surgical device for the treatment of open angle glaucoma through direct surgery.

About two percent of people in Europe and in the United States have glaucoma. Glaucoma is a group of eye diseases that can damage the eye's optic nerve and can cause corresponding visual field loss resulting in blindness if left untreated.

A considerable rise in intraocular pressure appears to be a major etiologic factor in glaucoma.

The eyeball is basically a rigid sphere filled with fluids. There are three chambers of fluid in the eye: Anterior Chamber (between the cornea and the iris), Posterior Chamber (between iris, zonule fiber and lens) and the Vitreous Chamber (between the lens and the retina). The first two chambers are filled with a clear fluid called aqueous humour whereas the vitreous chamber is filled with a more viscous fluid, the vitreous humour. The aqueous humour or "aqueous" carries nutrients to the lens and cornea, both of which have no blood supply. The aqueous is constantly secreted by the ciliary body (which is located behind the iris and around the lens) and flows from the posterior chamber through the pupil into the anterior chamber and drains out of the eye through a spongy tissue called trabecular meshwork (TM).

TM is located in the drainage angle of the anterior chamber, between the internal periphery of the cornea and the outer rim of the iris at the location where the iris meets the external wall of the eye (sclera). The fluid drains from the TM into a small canal (the Schlemm canal), then into aqueous collector channels and into aqueous veins.

Aqueous is produced by the ciliary body and removed from the eye at a constant rate to maintain a constant pressure in the anterior chamber of the eye. If the resistance to fluid flow increases, pressure inside the eye increases and the circulation of blood to the optic nerve is restricted. If the ocular pressure remains elevated for prolonged periods of time, the fibres of the optic nerve may cause atrophy resulting in loss of vision in the afflicted eye.

Glaucoma is roughly classified into two categories: closed angle glaucoma and open angle glaucoma. The closed angle glaucoma is caused by closure of angle of the anterior chamber by contact between the iris and the inner surface of the TM. Closure of this anatomical angle prevents normal drainage of aqueous from the anterior chamber. In open angle glaucoma the angle of the anterior chamber remains open, but the exit of aqueous through the TM is diminished. The source of resistance to outflow is in the TM.

Many of the current therapies for glaucoma are directed at reducing intraocular pressure. This is initially treated using medical therapy with drops or pills that reduce the production of aqueous or increase the outflow of aqueous. However, these various drug therapies for glaucoma are sometimes associated with considerable side effects, such as headaches, blurred vision, allergic reactions, cardiopulmonary complications and potential interactions with other drugs.

When the drug therapy fails, surgical therapy is used.

Surgical therapy for open angle glaucoma includes laser and surgery methods. The surgery methods can be classified as follows.

Goniotomy/trabeculotomy: These are simple and direct microsurgical dissection techniques with mechanical destruction of the TM. Initially this surgery provided favourable responses, however long term results showed only limited success in adults. These procedures probably failed later following repair mechanisms and "filling" processes. The "filling in" effect is the result of the healing process forming scars, which have the detrimental effect of collapsing and closing the opening that has been created in the TM. Once this opening is closed the pressure builds up inside once more and the surgery fails.

Goniocurettage: This is an ab-interno (performed from the inside) mechanical destructive technique. An instrument similar to a cyclodialysis spatula is used with a microcurette on the tip. Initial results are similar to trabeculotomy with subsequential failure following repair mechanisms and the filling in process.

Trabeculectomy: This is the most commonly performed filtering surgery. It involves creating a tiny filtering valve in the sclera. This procedure controls pressure by creating a new drainage channel through the angle structures to the extracellular space beneath the conjunctiva.

Trabeculectomy is a major surgery and is aided with locally applied anticancer drugs such as 5-flurouracil or mitomycin-c to reduce scarring and increase surgical success. Current mortality associated with trabeculectomy consists of failure (10-15%), infections (a life long risk about 2-5%), choroidal haemorrhage (1%, a severe internal haemorrhage from insufficient pressure resulting in visual loss), cataract formation, and hypotony maculopathy (potentially reversible visual loss from insufficient pressure). Another disadvantage of this procedure is that the body's natural healing process may gradually close the filter, causing the pressure to rise again.

Viscocanulostomy (VC) and Non Penetrating Trabeculectomy (NPT) are two new variations of filtering surgery. These are both major surgery procedures in which the Schlemm canal is surgically exposed by making a large and very deep scleral flap. In the VC procedure, the Schlemm canal is canulated and a viscoelastic substance injected (which dilates the Schlemm canal and aqueous collector channels). In the NPT procedure, the inner wall of the Schlemm canal is stripped away after the canal has been surgically exposed.

Trabeculectomy, VC and NPT are performed under a conjunctival and scleral flap so that the aqueous is drained onto the surface of the eye or into the tissues located near the lateral wall of the eye. Normal physiological outflows are not used.

Drainage devices: When Trabeculectomy, VC and NPT are not considered to have good probabilities of success, a number of implantable drainage devices are used to ensure that the desired filtration and outflow of aqueous through the surgical opening can continue. Placing glaucoma drainage implants also increases the risk of haemorrhage, infection and postoperative double vision that is a complication unique to drainage implants.

The treatment procedures and variations described above have numerous disadvantages and are generally only moderately successful. They involve considerable trauma to the eye and require great surgical skill to create a hole in the total thickness of the sclera/cornea in the sub-conjunctival space. Furthermore, normal physiological outflow pathways are not used. Procedure is lengthy and requires an operating theatre and the presence of an anaesthesiologist with all the costs involved, and the vision recovery time is also a long process.

The complications of filtration surgery have induced ophthalmic surgeons to look into alternative approaches for lowering intraocular pressure. The TM and the juxtacanalicular tissues both create the main resistance to aqueous outflow and for this reason they are the logical targets for surgical treatment of open angle glaucoma. Trabecular surgery has a much lower potential risk of choroidal haemorrhage, infections and furthermore, it aims at restoring physiologic outflow mechanisms. This surgery can be performed under local anaesthesia with rapid visual recovery.

WO-A-2005/070490 discloses a microcannula having a rounded distal tip. The rounded tip is used in conjunction with a light emitting signaling beacon such that the light is delivered proximal to the rounded tip, in order to disperse the light.

WO 2006/066103 A2 discloses a delivery instrument with fiber optics. The instrument has features to aid attachment and release of the implant.

The aim of the present invention is to eliminate at least one of the disadvantages of the prior art. More specifically, the invention aims at providing an ophtalmic surgical device for the efficacious treatment of open angle glaucoma.

### The invention is defined by the claims.

For example the device according to the invention comprises a fiber having a proximal end and a distal end, wherein the fiber is provided, near its proximal end, with a light source, whereby the distal end of the fiber forms a rounded tip, wherein the fiber has substantially constant diameter that is smaller than the diameter of the rounded tip.

The disclosure also relates to a method of performing ophthalmic surgery, comprising the steps of forming an incision in the eye's Schlemm's canal, inserting a fiber's distal end through the incision into the Schlemm's canal, advancing the fiber's distal end around in the Schlemm's canal, attaching a suture to the fiber's distal end, retracting the fiber backwardly through the Schlemm's canal, and connecting the suture ends.

By drawing a suture through the Schlemm's canal and connecting the suture ends, in an elegant way, the canal can be opened re-establishing a natural outflow and reducing the intraocular eye over pressure. As a result, an efficacious treatment of open angle glaucoma is obtained. In an advantageous way, the suture can be drawn through the Schlemms's canal by attaching the suture to the notch a the fiber's distal end, thereby counteracting additional injury during the process of retracting the fiber backwardly.

Further advantageous embodiments according to the invention are described in the following claims.

By way of example only, embodiments of the present invention will now be described with reference to the accompanying figures in which
Fig. 1 shows a schematic cross sectional view of a first embodiment of an ophthalmic surgical device according to the invention;
Fig. 2 shows a schematic partial cross sectional view of the human eye;
Fig. 3a shows a schematic view of the device in a first state;
Fig. 3b shows a schematic view of the device in a second state;
Fig. 3c shows a schematic view of the device in a third state;
Fig. 4 shows a schematic partial cross sectional view of a second embodiment of an ophthalmic surgical device according to the invention; and
Fig. 5 shows a flow chart of steps of a method.

It is noted that the figures show merely a preferred embodiment according to the invention. In the figures, the same reference numbers refer to equal or corresponding parts.

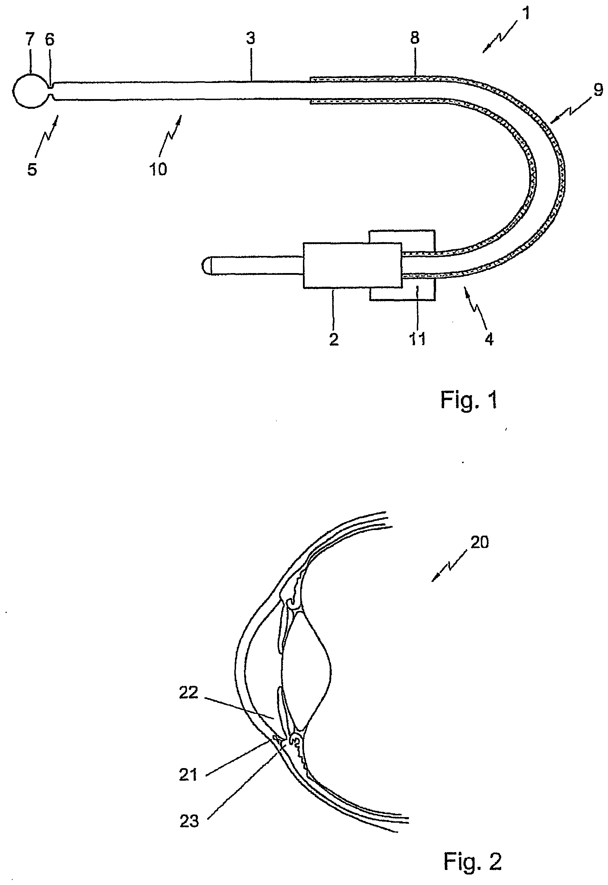

Figure 1 shows a schematic cross sectional view of a first embodiment of an ophthalmic surgical device 1 according to the invention. The surgical device 1 is intended for use in a surgical method for treatment of glaucoma, as explained in more detail below.

The device 1 comprises a fiber connector 2 and a fiber 3. The fiber 3 has a proximal end 4 and a distal end 5. The proximal end 4 is connected to the fiber connector 2, while the fiber 3 is further provided, near its distal end, with a notch 6. The distal end 5 of the fiber forms a rounded tip 7 to minimize tissue trauma and allow the fiber to advance in small tissue spaces, such as the eye's Schlemm's channel. In addition, light propagating through the fiber 3 is dispersed by the rounded tip 7, thereby visualizing the channel's interior under a range of incidence angles.

The fiber 3 has a substantially constant diameter, advantageously less than circa 0.2 mm, e.g. circa 0.15 mm. Preferably, the fiber diameter is smaller than the diameter of the rounded tip 7, thus further facilitating movement of the fiber through Schlemm's channel. As an example, the rounded tip diameter is circa 0.2 mm.

As shown in Fig. 1, the notch 6 is located close to the rounded tip 7, just behind the tip 7. In principle, the notch 6 can also be realized at a location offset from the tip 7. However, in order to draw a suture through the Schlemm's channel properly, the offset between the tip 7 and the notch 6 is relatively small.

The device 1 further comprises an optional coating layer 8 covering a proximal end section 9 the fiber 3. The coating layer 8 provides mechanical protection to the fiber 3. A distal end section 10 of the fiber 3 is free of a coating layer 8 to minimize its radial thickness. As an example, the distance of the free distal end section 10 is circa 40 cm. However, also other distances can be applied, e.g. more than 40 cm such as circa 50 cm, or less than 40 cm such as circa 30 cm. The device 1 according to the invention may have a total length in a range between circa 50 cm and circa 250 cm, e.g. 2 m, 1 m or 50 cm. The total length of the fiber may be shorter, e.g. between circa 10 cm and 100 cm, e.g. circa 30 cm.

In the transition area between the fiber proximal end 4 and the fiber connector 2, a silicone layer 11 is provided on top of the connector exterior surface and the coating layer 8.

Figure 2 shows a schematic partial cross sectional view of the human eye 20. The eye contains a so-called canal of Schlemm 21 for flowing aqueous humour from the anterior chamber 22 into aqueous collector channels 23 and into aqueous veins.

According to an aspect of the disclosure treatment of open angle glaucoma is performed through direct surgery, using the ophthalmic surgical device 1 shown in Fig. 1.

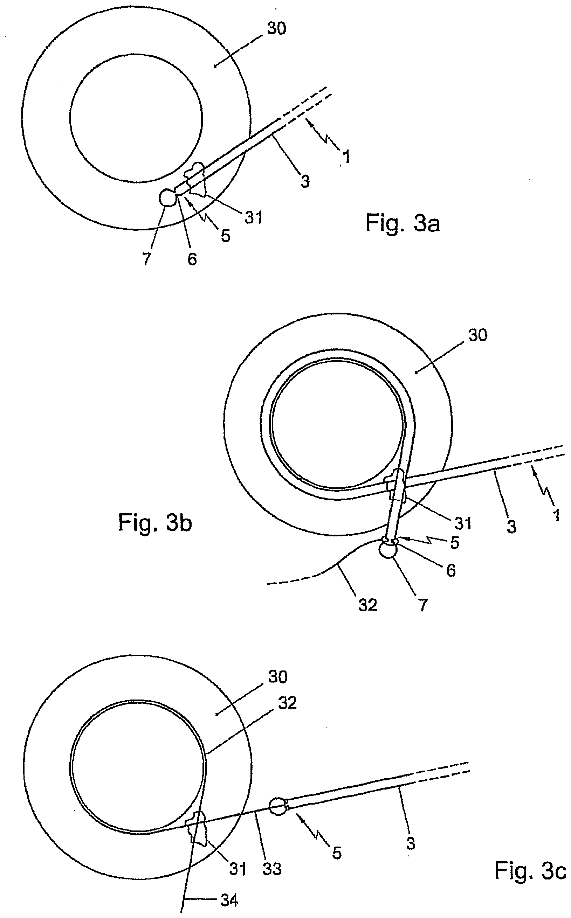

Figures 3a,b,c show a schematic view of the device 1 in a first, second and third state, respectively. In the process of treating the glaucoma, an incision is formed in the eye's Schlemm's canal 30. By making the incision, a trabeculectomy flap 31 is created for opening the canal 30. Then, the fiber's distal end 5 is inserted through the incision 31 into the canal 30, as shown in Fig. 3a. The fiber's distal end 5 is advanced in the Schlemm's canal 30, all around, and protrudes through the same incision 31, as shown in Fig. 3b. Subsequently, a suture 32 is attached to the neck of the distal end, formed by the rounded end tip 7 and the notch 6 behind the tip 7. Then, the fiber 3 is retracted backwardly through the Schlemm's canal 30 until the fiber 3 is removed from the canal 30 and the suture 32 is thus drawn all around in the canal 30. The suture 32 can be released from the fiber 3 so that the suture ends 33, 34 can now be connected, preferably with a little tension to stretch and open the Schlemm's canal 30.

By advancing the fiber's distal end 5 over 360°, the incision 31 can be used both for inserting the tip 7 into and from the canal 30, thereby reducing additional trauma. The suture 32 can be manufactured from a variety of materials, e.g. from 10,0 prolyne.

According to an aspect of the invention, the fiber 3 is illuminated while advancing the fiber's distal end 5 in the Schlemm's canal 30, thereby facilitating the advancing process since the person operating the ophthalmic surgical device 1 can easily keep track of progress of the fiber 3 in the canal 30. Thereto, the fiber connector 2 is connected to a light source generating light that propagates through the fiber 3.

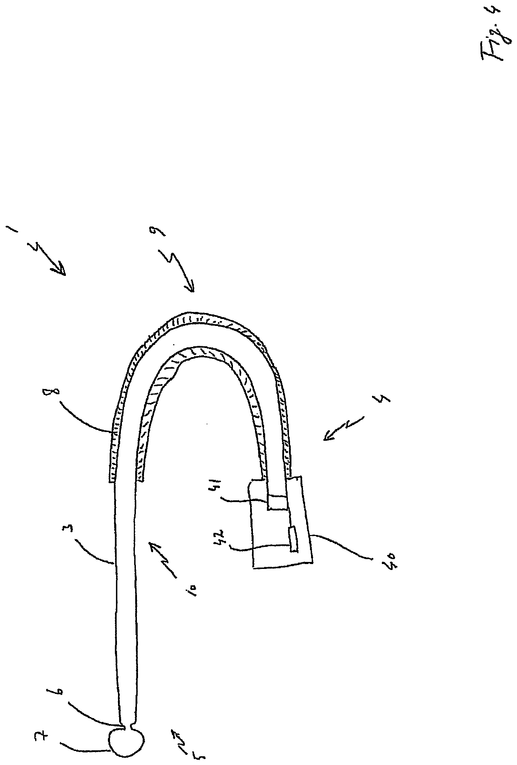

Figure 4 shows a schematic partial cross sectional view of a second embodiment of an ophthalmic surgical device 1 according to the invention. Here, instead of the fiber connector 2 shown in Fig. 1, the fiber 3 in the device 1 according to the second embodiment is further provided, near its proximal end, with a light source 40. In the shown embodiment, the light source is integrated with the fiber's distal end. Further, the light source comprises a LED 41 and an energy source, such as a battery 42, for feeding the light source. In principle, also another light source can be used, such as a halogen light source. Alternatively, the light source can be implemented as a disposable unit, e.g. by designing the light source as a separate unit that can be mounted to the fiber 3, e.g. using a snap connection.

By applying a light source on the proximal end of the fiber, the device can advantageously be used without an external light source, thereby increasing the ease of use of the device. Also, the fiber may have a shorter length, thereby further increasing the ease of use of the device, e.g. in providing the user of the device with an overview including less complicated structures.

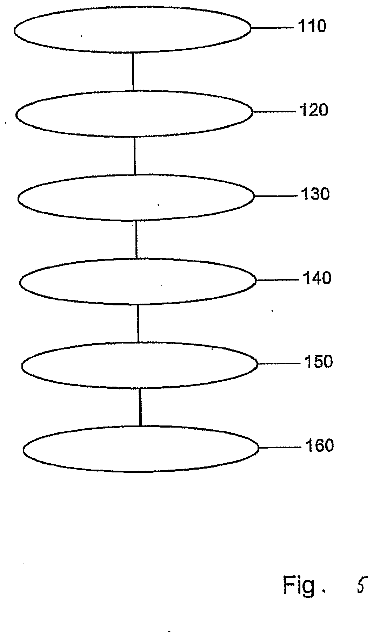

Figure 5 shows a flow chart of method steps. The method includes performing ophthalmic surgery. The method comprises the step 110 of forming an incision in the eye's Schlemm's canal, a step 120 of inserting a fiber's distal end through the incision into the Schlemm's canal, a step 130 of advancing the fiber's distal end around (360 graden, all around) in the Schlemm's canal, a step 140 of attaching a suture to the fiber's distal end, a step 150 of retracting the fiber backwardly through the Schlemm's canal, and a step 160 of connecting the suture ends.

The invention is not restricted to the embodiments described herein. It will be understood that many variants are possible.

These and other embodiments will be apparent for the person skilled in the art and are considered to lie within the scope of the invention as defined in the following claims.

## Claims

1. An ophthalmic surgical device (1) for the treatment of glaucoma, comprising a fiber connector (2) and a fiber (3) having a proximal end (4) and a distal end (5), the proximal end (4) connected to the fiber connector, wherein the fiber is provided, near its distal end, with a notch (6) close to a rounded tip (7) formed by the distal end of the fiber.

2. An ophthalmic surgical device according to claim 1, wherein the fiber (3) has a substantially constant diameter that is smaller than the diameter of the rounded tip.

3. An ophthalmic surgical device according to any of the preceding claims, wherein the notch (6) is configured to draw a suture through the Schlemm's channel.

4. An ophthalmic surgical device according to any of the preceding claims, wherein the substantially constant diameter of the fiber (3) is smaller than circa 0.2mm.

5. An ophthalmic surgical device according to any of the preceding claims, wherein a distal end section of the fiber is free of a coating layer.

## Patentansprüche

1. Augenoptische chirurgische Vorrichtung (1) zur Behandlung von Glaukom, umfassend einen LWL-Steckverbinder (2) und eine Faser (3) mit einem proximalen Ende (4) und einem distalen Ende (5), wobei das proximale Ende (4) mit dem LWL-Steckverbinder verbunden ist, wobei die Faser nahe ihres distalen Endes mit einer Kerbe (6) nahe einer abgerundeten Spitze (7) bereitgestellt ist, die durch das distale Ende der Faser gebildet wird.

2. Augenoptische chirurgische Vorrichtung nach Anspruch 1, wobei die Faser (3) einen im Wesentlichen konstanten Durchmesser aufweist, der kleiner ist als der Durchmesser der abgerundeten Spitze.

3. Augenoptische chirurgische Vorrichtung nach einem der vorstehenden Ansprüche, wobei die Kerbe (6) so gestaltet ist, dass sie einen Faden durch den Schlemm-Kanal zieht.

4. Augenoptische chirurgische Vorrichtung nach einem der vorstehenden Ansprüche, wobei der im Wesentlichen konstante Durchmesser der Faser (3) kleiner ist als etwa 0,2 mm.

5. Augenoptische chirurgische Vorrichtung nach einem der vorstehenden Ansprüche, wobei ein distales Endstück der Faser frei ist von einer Überzugsschicht.

## Revendications

1. Dispositif chirurgical ophtalmique (1) pour le traitement du glaucome, comprenant un connecteur de fibre (2) et une fibre (3) ayant une extrémité proximale (4) et une extrémité distale (5), l'extrémité proximale (4) étant connectée au connecteur de fibre, la fibre étant pourvue, près de son extrémité distale, d'une encoche (6) proche d'une pointe arrondie (7) formée par l'extrémité distale de la fibre.

2. Dispositif chirurgical ophtalmique selon la revendication 1, la fibre (3) ayant un diamètre sensiblement constant qui est plus petit que le diamètre de la pointe arrondie.

3. Dispositif chirurgical ophtalmique selon l'une quelconque des revendications précédentes, l'encoche (6) étant conçue pour tirer un fil de suture à travers le canal de Schlemm.

4. Dispositif chirurgical ophtalmique selon l'une quelconque des revendications précédentes, le diamètre sensiblement constant de la fibre (3) étant inférieur à environ 0,2 mm.

5. Dispositif chirurgical ophtalmique selon l'une quelconque des revendications précédentes, une section d'extrémité distale de la fibre étant exempte de couche de revêtement.
